# EUROPEAN PATENT SPECIFICATION

(11) **EP 3 306 319 B1**
(45) Date of publication and mention of the grant of the patent: **25.03.2020**
(21) Application number: 16803232.4
(22) Date of filing: 27.05.2016
(51) Int. Cl.: G01N 33/569, C07K 16/12, G01N 33/543, G01N 33/577, C12N 15/02, C12P 21/08

(54) **TEST OBJECT DETECTION METHOD, AND IMMUNOASSAY INSTRUMENT AND MONOCLONAL ANTIBODY FOR SAME**
TESTOBJEKTDETEKTIONSVERFAHREN SOWIE IMMUNOASSAY-INSTRUMENT UND MONOKLONALER ANTIKÖRPER DAFÜR
PROCÉDÉ DE DÉTECTION D'OBJET D'ESSAI, ET INSTRUMENT DE DOSAGE IMMUNOLOGIQUE ET ANTICORPS MONOCLONAL ASSOCIÉS

(30) Priority: 29.05.2015 JP 2015110585
(43) Date of publication of application: 11.04.2018
(73) Proprietor: Denka Seiken Co., Ltd., Chuo-ku Tokyo 103-8338 (JP)
(72) Inventor: KOHIYAMA, Risa, Gosen-shi Niigata 959-1834 (JP); MIYAZAWA, Takashi, Gosen-shi Niigata 959-1834 (JP)
(74) Representative: Mewburn Ellis LLP
(86) International application number: PCT/JP2016/065683
(87) International publication number: WO 2016/194797

(56) References cited:
- WO-A1-88/04669
- WO-A1-2015/025968
- JP-A- 2004 258 024
- JP-A- 2011 079 830
- JP-A- 2011 220 931
- GUTTIKONDA, SUJATHA ET AL.: 'Monospecific and bispecific antibodies against E. coli 0157 for diagnostics' JOURNAL OF IMMUNOLOGICAL METHODS vol. 327, no. 1-2, 2007, pages 1 - 9, XP022266385
- OWAIS, MOHAMMAD ET AL.: 'An Alternative Chemical Redox Method for the Production of Bispecific Antibodies: Implication in Rapid Detection of Food Borne Pathogens' PLOS ONE vol. 9, no. 3, 2014, pages 1 - 13, XP055333846

## Description

### TECHNICAL FIELD

The present invention relates to a method of detecting a test subject such as a pathogen by an immunoassay, and an immunoassay instrument and a monoclonal antibody therefor.

### BACKGROUND ART

Since monoclonal antibodies recognize only a specific antigen, they are widely used for the detection of the specific antigen. For example, an immunoassay of *Mycoplasma pneumoniae,* which immunoassay employs a sandwich method in which a monoclonal antibody that recognizes P30 protein of *Mycoplasma pneumoniae* is immobilized on a solid phase, is described in Patent Document 1.

### PRIOR ART REFERENCES

### PATENT DOCUMENTS

Patent Document 1: WO 2015/025968

JP 2011/220931 discloses an immuno-chromatography reagent for detection of *Mycoplasma pneumoniae* antigens.

JP 2004/258024 discloses an immunoassay for diagnosing and identifying dermatophytes.

JP 2011/079830 discloses an antibody directed to an antigen of *Mycoplasma pneumoniae.*

### SUMMARY OF THE INVENTION

### PROBLEMS TO BE SOLVED BY THE INVENTION

A polyclonal antibody is a mixture of various antibodies which recognize various antigens or antigenic determinant regions, while a monoclonal antibody recognizes only a certain region of a certain antigen. Because of this property, a monoclonal antibody generally exhibits a higher specificity than a polyclonal antibody. On the other hand, it is easily assumed that a monoclonal antibody is inferior in the kinds and total number of antigens which can bind to the antibody as compared to a polyclonal antibody. This results in low sensitivity of a monoclonal antibody as well as an immunoassay and an immunoassay instrument using it.

An object of the present invention is to provide a novel method of detecting a test subject by an immunoassay, in which low sensitivity due to using a monoclonal antibody is improved in spite of using a monoclonal antibody in the immunoassay. Another object of the present invention is to provide an immunoassay instrument for the detection method of the present invention. Still another object of the present invention is to provide a novel monoclonal antibody which can be used for the detection of *Mycoplasma pneumoniae* by the detection method of the present invention.

### MEANS FOR SOLVING THE PROBLEMS

In order to compensate the disadvantage of low sensitivity of a monoclonal antibody, an immunoassay using multiple monoclonal antibodies is conceivable. In a general immunoassay such as ELISA or immunochromatography, the amount of the antibodies which can be used depends on the area of an ELISA plate or an immunochromatographic strip or the area of a labeled substance. In this case, the use of multiple monoclonal antibodies is superior in the kinds and total number of antigens which can bind to the antibodies as compared to the use of a single monoclonal antibody. Although there is no appreciable difference when the amount of antigens is excess to the amount of the antibodies used, the sensitivity in an immunoassay is improved when the amount of antigens is insufficient for the amount of the antibodies used.

However, the amount of each monoclonal antibody used is less than the amount of a single monoclonal antibody used in cases where a single monoclonal antibody is used. As a result, probability of encounter with an antibody per a certain antigen is decreased, so that reactivity per unit time is decreased. This is one of the factors that make it difficult to accomplish the improvement of the sensitivity in an immunoassay and this has made it difficult to obtain satisfactory sensitivity.

The present inventors intensively studied to discover that a method using a monoclonal antibody which recognizes two or more kinds of antigens while having specificity is superior to a method using a single or multiple conventional monoclonal antibodies in sensitivity, thereby completing the present invention, which is defined in the claims. Furthermore, the present inventors discovered that an immunoassay using this monoclonal antibody is superior to an immunoassay using a conventional monoclonal antibody in sensitivity, thereby completing the present invention, as defined in the claims.

That is, the present invention provides a method of detecting *Mycoplasma pneumoniae* having two or more kinds of antigens which enable detection of the *Mycoplasma pneumoniae*, the method comprising detecting the *Mycoplasma pneumoniae* by an immunoassay using a monoclonal antibody or an antigen-binding fragment thereof which recognizes the two or more kinds of antigens, wherein said two or more kinds of antigens are P1 protein and P30 proteins. The present invention also provides an immunoassay instrument for carrying out the method of the present invention, comprising a solid phase on which the monoclonal antibody or an antigen-binding fragment thereof is immobilized, as set out in the claims. The present invention further provides a monoclonal antibody which specifically reacts with P1 protein and P30 protein derived from *Mycoplasma pneumoniae.*

### EFFECT OF THE INVENTION

In the method of the present invention, a monoclonal antibody is used for an immunoassay so that the specificity is high, while low sensitivity due to the use of a monoclonal antibody is improved. By the present invention, an immunoassay instrument and a monoclonal antibody used for the novel detection method of the present invention are also provided.

### BRIEF DESCRIPTION OF THE DRAWINGS

Figure 1 is a diagram showing the results of examining the reactivities of the monoclonal antibodies of the present invention prepared in the Examples below by Western blotting.
Figure 2 is a schematic diagram of one preferred embodiment of the immunochromatographic immunoassay instrument of the present invention.

### BEST MODE FOR CARRYING OUT THE INVENTION

The test subject described herein is a test subject having two or more kinds of antigens which enable detection of the test subject. Examples of such a test subject include, but not limited to, a variety of pathogens such as bacteria, viruses, fungi and rickettsias. In the following examples, the test subject is *Mycoplasma pneumoniae* and the two or more kinds of antigens are P1 protein and P30 protein. It should be noted that even if the test subject is quantified or semiquantified, quantification and semiquantification are included in "detection" as used in the present invention because they inevitably accompany "detection".

In the method of the present invention, an immunoassay is performed using a monoclonal antibody or an antigen-binding fragment thereof which recognizes the above-mentioned two or more kinds of antigens. Here, "recognize(s)" refers to that the antibody specifically reacts, i.e., undergoes antigen-antibody reaction. "Specifically" means that in a liquid system in which a protein and an antibody thereof are mixed together, the antibody does not cause an antigen-antibody reaction with the protein component which is an antigen at a detectable level, or merely causes obviously weaker reaction than the antigen-antibody reaction of the antibody with the corresponding antigen thereof even if it causes some binding reaction or association reaction.

An antigen-binding fragment obtained by separating only the antigen binding site from the monoclonal antibody of the present invention can be used in the method of the present invention. That is, cases where a fragment having a specific antigen-binding property (an antigen-binding fragment) such as a Fab, a Fab', a F(ab')₂ or a single-chain antibody (scFv) that is produced by a known method is used are within the scope of the present invention. Further, the class of the monoclonal antibody is not limited to IgG, and may be IgM or IgY.

The monoclonal antibody used in the method of the present invention can be obtained by immunizing an animal to be immunized with a composite or extract having two or more kinds of antigens of interest, or one antigen of the two or more kinds of antigens or a partial peptide thereof by a known immunologic technique and preparing a hybridoma using cells of the immunized animal. Although the length of the peptide used in the immunization is not particularly limited, a peptide having preferably 5 or more amino acids, more preferably 10 or more amino acids can be used as an immunogen. The immunogen can be obtained from a culture solution, or obtained by incorporating a DNA encoding an arbitrary antigen into a plasmid vector and introducing it into a host cell to express. An arbitrary antigen or a partial peptide thereof used as an immunogen can be expressed as a fusion protein with a protein exemplified below and used as an immunogen after or without purification. Glutathione S-transferase (GST), maltose binding protein (MBP), thioredoxin (TRX), Nus tag, S tag, HSV tag, FRAG tag, polyhistidine tag and the like, which are commonly used as "protein expression/purification tags" by those skilled in the art can be used in the preparation of the fusion protein. Preferably, the fusion protein with the tag is used as an immunogen after it is cleaved to the arbitrary antigen or a partial peptide thereof and the other tag portion by a digestive enzyme, and after separation and purification.

The preparation of the monoclonal antibody from the immunized animal can be easily performed by the well-known method of Kohller et al. (Kohler et al., Nature, vol, 256, p495-497(1975)). That is, antibody producing cells such as splenocytes and lymphocytes are collected from the immunized animal and fused with mouse myeloma cells to produce hybridomas in the usual manner. The resulting hybridomas are cloned by a limiting dilution method or the like. Then, monoclonal antibodies that undergo antigen-antibody reaction with the antigen used in immunizing the animal are selected from the monoclonal antibodies produced from each cloned hybridoma.

Since the monoclonal antibody used in the method of the present invention recognizes two or more kinds of antigens, the monoclonal antibodies selected in this way, which recognize one of the two or more kinds of antigens, are screened for a monoclonal antibody that recognizes another antigen. In cases where a monoclonal antibody that recognizes three or more kinds of antigens is used, similar screening is further repeated to select a monoclonal antibody that recognizes each antigen. The method of the present invention uses a monoclonal antibody or an antigen-binding fragment thereof which recognizes both of two or more kinds of antigens obtained in this way. In order that a monoclonal antibody that recognizes two kinds of antigens can exist, there are various cases that can be considered such as a case wherein the monoclonal antibody recognizes regions having high homology within each of two different antigens, a case wherein the monoclonal antibody recognizes regions having a similar conformation within each of different antigens, a case wherein the monoclonal antibody recognizes a region spanning the adjacent areas of the composite composed of different antigens, and a case wherein the monoclonal antibody recognizes a conformation spanning the adjacent areas of the composite composed of different antigens. Thus, "a method of detecting a test subject having two or more kinds of antigens which enable detection of the test subject" as used herein is limited to the cases where a monoclonal antibody that recognizes two or more kinds of antigens is obtained. Furthermore, the monoclonal antibody preferably recognizes two kinds of antigens. This is because in general, as the number of kinds of antigens recognized increases, it becomes difficult to obtain a monoclonal antibody that recognizes the antigens. In the following examples, the monoclonal antibody that recognizes P1 protein and P30 protein of *Mycoplasma pneumoniae* is obtained by such screening.

For the purification of a monoclonal antibody from an ascites or a culture supernatant, a known immunogloblin purification method can be used. Examples of the method include fractionation methods by salting out using ammonium sulfate or sodium sulfate, PEG fractionation methods, ethanol fractionation methods, DEAE ion exchange chromatography methods and gel filtration methods. It is also possible to perform the purification by an affinity chromatography method using a carrier to which any one of Protein A, Protein G and Protein L is bound depending on the species of the immunized animal and the class of the monoclonal antibody.

In the immunoassay of the present invention, the assay is performed by an immunoassay utilizing the antigen-antibody reaction of the monoclonal antibody or the antigen-binding fragment thereof which recognizes two or more kinds of antigens prepared as described above (hereinafter, in the descriptions before the examples, "antibody" refers to "antibody or antigen-binding fragment thereof' unless the context clearly dictates otherwise) with the antigen in the sample. Any method well known by those skilled in the art such as a competitive method, an agglutination method, a western blotting method, an immunostaining method or a sandwich method can be used as the immunoassay therefor. In the present invention, "assay" includes all of quantification, semiquantification and detection.

As the immunoassay, the sandwich method is preferable. The sandwich method itself is well known in the field of immunoassay and can be performed by an immunochromatography or ELISA. Each of these sandwich methods is well known and the method of the present invention can be performed according to a well-known sandwich method except that the monoclonal antibody of the present invention as described above, which recognizes two or more kinds of antigens, is used.

In a sandwich method, two kinds of antibodies that recognize antigens (an antibody immobilized on a solid phase and a labeled antibody) are used, and in the method of the present invention, at least one of the two kinds of antibodies is the monoclonal antibody which recognizes two or more kinds of antigens as described above. As described below, the monoclonal antibody which recognizes two or more kinds of antigens as described above is preferably used at least as the immobilized antibody in order to better accomplish the sensitivity improvement that is an object of the present invention, because the antibody immobilized on a solid phase is limited in the amount of the antibody which can be immobilized per area. In cases where at least two kinds of antigens recognized by the monoclonal antibody are present in a single molecule or a single composite, the sandwich method can be performed using a single kind of the monoclonal antibody as the immobilized antibody and the labeled antibody (See examples below).

In the immunoassay whose detection principle is the sandwich method, anything on which an antibody can be immobilized by a known method can be used as the solid phase on which the antibody is immobilized. For example, known one such as a porous thin film (membrane) with capillary action, a particulate matter, a test tube and a resin flat plate can be arbitrarily selected. As a substance labeling the antibody, an enzyme, a radioactive isotope, a fluorescent substance, a luminescent substance, a colored particle, a colloidal particle or the like can be used. Among the immunoassays using the above-mentioned various materials, a lateral flow immunoassay using a membrane is particularly preferable in view of simplicity and quickness of clinical testing.

The present invention also provides an immunoassay instrument that enables the immunoassay to be performed in lateral flow manner using the monoclonal antibody which recognizes two or more kinds of antigens. The immunoassay instrument provided by the present invention comprises a support having a detection region in which an antibody (antibody 1) that captures a target to be measured (antigen) is immobilized, a labeled substance region having a movable labeled antibody (antibody 2), a sample pad to which a sample is added dropwise, an absorption band which absorbs the developed sample liquid, and a backing sheet for attaching these members together, wherein at least one of the antibody 1 and the antibody 2 is the monoclonal antibody of the present invention which recognizes two or more kinds of antigens.

The number of the detection regions and the type of the labeled antibodies included in the labeled substance region are not limited to 1. By using antibodies corresponding to a plurality of targets to be measured, two or more kinds of antigens can be detected by the same immunoassay instrument.

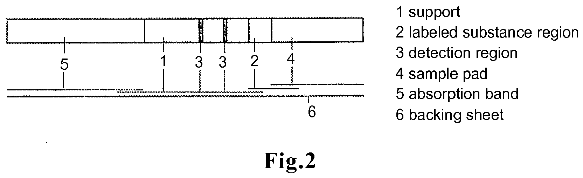

Figure 2 shows a preferred embodiment of the immunochromatographic immunoassay instrument of the present invention. The reference numeral 1 denotes a support, the numeral 2 denotes a labeled substance region, the numeral 3 denotes a detection region, the numeral 4 denotes a sample pad, the numeral 5 denotes an absorption band and the numeral 6 denotes a backing sheet.

The upper figure in Figure 2 is a top view and the lower figure is a cross-sectional view. In the example shown in the figures, the support in which two detection regions are formed, the absorption band, the labeled substance region and the sample pad are each laminated on the backing sheet. As shown in the figures, one end of the absorption band and one end of the support, the other end of the support and one end of the labeled substance region and the other end of the labeled substance region and one end of the sample pad are overlapped with each other so that a continuous lateral flow passage is formed.

The support is a material capable of immobilizing an antibody for capturing an antigen and has a performance that does not prevent a liquid from passing in a horizontal direction. Preferably, the support is a porous thin film having a capillary action, and is a material capable of transporting a liquid and components dispersed therein by absorption. The material forming the support is not particularly limited, and examples thereof include cellulose, nitrocellulose, cellulose acetate, polyvinylidene difluoride (PVDF), glass fiber, nylon, polyketone and the like. Among them, a thin film made of nitrocellulose is more preferable.

The labeled substance region is made of a porous base material containing a labeled antibody, and a glass fiber, a nonwoven fabric or the like generally used can be used as the material of the base material. In order to impregnate a large amount of the labeled antibody, the base material is preferably in the form of a pad having a thickness of about 0.3 mm to 0.6 mm.

The detection region refers to a part of the support on which the antibody that captures the antigen is immobilized. It is preferable to provide a detection region comprising at least one area on which a monoclonal antibody that recognizes two or more kinds of antigens is immobilized and further provide a detection region for detecting a labeled antibody in order to assist actual diagnoses.

The sample pad is a site for dropping a specimen or a sample prepared using a specimen, and is a porous material having a water absorption property. Commonly-used cellulose, glass fiber, nonwoven fabric or the like can be used as the material. In order to use a large amount of the sample in the immunoassay, the material is preferably in the form of a pad having a thickness of about 0.3 mm to 1mm. The sample pad and the above-mentioned labeled substance region are merely functional distinctions and need not necessarily be made of separate materials. That is, it is also possible that a partial area of the material set as the sample pad has the function of the labeled substance region.

The absorption band is a member for absorbing components that are supplied to the support and not involved in the reaction in the detection regions. As the material, a filter paper, a sponge or the like having high water retentivity made of a general natural polymer compound, synthetic polymer or the like can be used, but in order to promote development of the sample, one having high water absorbability is preferable .

The backing sheet is a member for attaching and fixing all the above-mentioned materials, i.e., the support, the sample pad, the labeled substance region, and the absorption band with a partial overlap. The backing sheet is not always necessary as long as these materials can be arranged and fixed at optimal intervals, but it is generally preferable to use the backing sheet in view of convenience in manufacturing and use.

In the immunoassay instrument in the embodiment described with reference to Fig. 2, the sample passes through a porous flow passage formed by a series of connections of the sample pad, the labeled substance region, the support, the detection regions, and the absorption band. Therefore, in the present embodiment, all of them are sample moving regions. Depending on the quality and form of each constituent material, there may be a mode in which the sample does not penetrate the interior of the material and pass through the interface. However, since it does not matter whether the sample movement region defined in this specification is in the interior or the interface of the material, an immunoassay instrument in such a mode is also included within the scope of this specification.

Based on the embodiment of Fig. 2, a method of using the immunoassay instrument of the present invention will be described. Measurement is initiated by dropping a specimen or a sample prepared using a specimen onto the sample pad. It is preferable that the sample to be dropped be previously diluted about 2 to 20-fold with a buffer containing a surfactant.

The sample dropped onto the sample pad is developed in the horizontal direction sequentially to the labeled substance region, the support and the absorption band by capillary action. In the labeled substance region, the labeled antibody is released into the solution and developed to the support together with the development of the sample. In cases where an antigen is present in the sample, the antigen is specifically captured by a capture antibody in the detection regions of the support, and the antigen also forms a complex by a specific reaction with the labeled antibody. In this way, a sandwich of the antibodies via the antigen is established in the detection regions, and the labeled antibody-antigen complex can be detected in the detection regions.

As a monoclonal antibody that recognizes two or more kinds of antigens while having specificity, there are various antibodies that can be considered such as a monoclonal antibody that recognizes a region having high homology within each of different antigens, a monoclonal antibody that recognizes a region having a similar conformation within each of different antigens, a monoclonal antibody that recognizes a region spanning the adjacent areas of the composite composed of different antigens and a monoclonal antibody that recognizes a conformation spanning the adjacent areas of the composite composed of different antigens, but it is more preferred that two or more kinds of antigens recognized by a single monoclonal antibody be included in the same composite. It should be noted that, here, "composite" refers to a substance in which a plurality of molecules are aggregated by some action in a specimen. For example, in cases where the test subject is a bacterial cell, each antigen on the bacterial cell can be considered to form a composite. In the following examples, it is considered that P1 protein and P30 protein of *Mycoplasma pneumoniae* form a "composite" as used herein.

Owing to the antigens included in the same composite, the possibility of receiving steric hindrance between antibodies is reduced and the probability of establishing a sandwich in the above-mentioned immunoassay is increased as compared with a case in which the antigens are present in a single substance.

The principle of improving the sensitivity of the immunoassay by the method of the present invention is considered as follows. In a sandwich method, an antibody immobilized on a solid phase is used, but the amount of the antibody that can be immobilized per unit area of the solid phase is limited. Also, the time of antigen-antibody reaction is limited. In particular, in an immunochromatography method, since the antigen-antibody reaction occurs only while a specimen or a sample prepared using a specimen (a dilution of a specimen or the like) flows from the upstream and passes through the detection regions, an antigen which cannot be bound to the antibody within this time flows to the downstream of the detection regions as it is and is not detected. When the amount of an antigen in the sample is small, the use of a monoclonal antibody that recognizes two or more kinds of antigens as an immobilized antibody improves the sensitivity more than the use of one kind of monoclonal antibody that recognizes one kind of antigen as the immobilized antibody (ordinary sandwich method), because the amount of the antigen bound to the antibody is increased. In addition, when two kinds of monoclonal antibodies each recognizing one kind of antigen are used in combination as the immobilized antibodies, the amount of each monoclonal antibody immobilized is each half of the total amount of the immobilized antibodies. Therefore, when the time of the antigen-antibody reaction is short, the probability that an antigen collides with and binds to a monoclonal antibody capable of binding to this antigen in a prescribed direction is 1/2 as compared with a case where the entire immobilized antibody is the monoclonal antibody that recognizes the antigen. On the other hand, when a monoclonal antibody that recognizes two or more kinds of antigens is used, any of two or more kinds of antigens will bind to the antibody if it collides with the immobilized antibody in a prescribed direction. Therefore, the amount of the antigens captured by the immobilized antibody is larger than that in cases where two kinds of monoclonal antibodies each recognizing one kind of antigen are used in combination, and thus the sensitivity of the immunoassay is improved.

### EXAMPLES

Hereinafter, the present invention will be described more specifically by way of examples thereof. However, the present invention is not limited to the following examples.

### Example 1 Preparation of Anti-Mycoplasma pneumoniae Monoclonal Antibody

### 1. Preparation of Mycoplasma pneumoniae Antigen

*Mycoplasma pneumoniae* was cultured and the culture solution inactivated by heat treatment at 60°C for 30 minutes was used.

### 2. Preparation of Anti-Mycoplasma pneumoniae Monoclonal Antibody

A BALB/c mouse was immunized with the mycoplasma inactivated antigen as described in section 1, and the spleen was excised from the mouse bred for a certain period of time and fused with mouse myeloma cells (P3 × 63) by the method of Kohler et al. (Kohler et al., Nature, vol 256, p 495-497 (1975)). The obtained fused cells (hybridomas) were maintained in an incubator at 37°C, and cell purification (monocloning) was performed while confirming the antibody activity of the supernatant by ELISA using a plate in which a *Mycoplasma pneumoniae* P1 antigen was immobilized and a plate in which a *Mycoplasma pneumoniae* P30 antigen was immobilized.

As a result, as shown in Table 1, a plurality of hybridoma cell lines producing *anti-Mycoplasma pneumoniae* P1 antibodies, *anti-Mycoplasma pneumoniae* P30 antibodies and anti-*Mycoplasma pneumoniae* P1-P30 antibodies were obtained.

**[Table 1]**

| Clone name | Reactive antigen |
|---|---|
| P1A | P1 |
| P1B | P1 |
| P30A | P30 |
| P30B | P30 |
| P1-P30A | P1, P30 |
| P1-P30B | P1, P30 |

P1 and P30 used in this ELISA were prepared by gel filtration and ion exchange chromatography.

The obtained cell lines were intraperitoneally administered to a pristane-treated BALB/c mouse, and about 2 weeks later, antibody-containing ascitic fluid was collected. IgG was purified from the obtained ascitic fluid by an affinity chromatography using a protein A column, and a plurality of purified anti-*Mycoplasma pneumoniae* monoclonal antibodies were obtained.

In the following examples, among a plurality of the obtained anti-*Mycoplasma pneumoniae* monoclonal antibodies, antibodies selected in consideration of reactivities and epitopes were used.

### Example 2 Reactivity of Anti-Mycoplasma pneumoniae Monoclonal Antibody

The reactivity between each monoclonal antibody obtained in Example 1 and *Mycoplasma pneumoniae* was confirmed by western blotting. *Mycoplasma pneumoniae* was cultured in PPLO Broth and the culture solution was used as a sample.

The culture solution of *Mycoplasma pneumoniae* and molecular weight markers were electrophoresed by a conventional SDS-PAGE. After the electrophoresis, the resultant was transferred to a PVDF membrane. After blocking with skim milk, the resultant was thoroughly washed with PBS-Tween. The anti-P30 antibody adjusted to 1 µg/mL with PBS-Tween was reacted at room temperature for 1 hour. After thorough washing with PBS-Tween, an HRP-labeled anti-mouse antibody diluted 3000-fold was reacted at room temperature for 1 hour. After thorough washing with PBS-Tween, the signal was detected using ECL reagent (GE Healthcare) or POD Immunostain Kit (Wako).

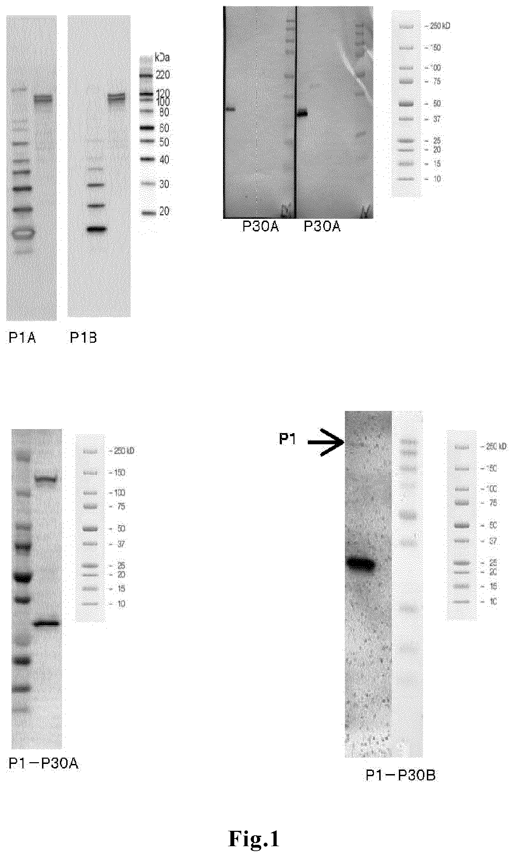

The results of the western blotting are shown in Fig. 1. As shown in Fig. 1, it was confirmed that the monoclonal antibodies obtained in Example 1 specifically react with each antigen similarly to the result of ELISA. The upper left figure of Fig. 1 (fluorescence detection in ECL) shows the results for P1A and P1B, and each right lane shows the result for the bacterial cells and the lane on the left shows the molecular weight markers (the same is true for the other figures in FIG. 1). The band of the P1 protein is clear in each right lane. The upper right figure of Fig. 1 (taken with a densitometer after POD staining) shows the results for P30A and P30B. The band of the P30 protein is clear in each left lane. The bottom left figure of Fig. 1 (taken with a densitometer after POD staining) shows the result for P1-P30A. In the left lane, the band of the P1 protein and the band of the P30 protein are clear. The bottom right figure of Fig. 1 (fluorescence detection by ECL reagent) shows the result for P1-P30B. The band of the P30 protein is clear, and the band of the P1 protein can be confirmed even though weak.

### Example 3 Immunoassay instrument for Measuring Mycoplasma pneumoniae

### 1. Immobilization of Anti-Mycoplasma pneumoniae Antibody on Nitrocellulose Membrane

A solution in which the antibody prepared in Example 1 was diluted to a concentration of 1.0 mg/mL with purified water, and an anti-mouse IgG antibody were prepared. The antibody and the anti-mouse IgG antibody were applied linearly on the sample pad side and the absorption body side of a nitrocellulose membrane lined with a PET film, respectively. Thereafter, the nitrocellulose membrane was dried at 45°C for 30 minutes to obtain an *anti-Mycoplasma pneumoniae* antibody-immobilized membrane. In this example, it is referred to as an antibody-immobilized membrane.

### 2. Immobilization of Anti-Mycoplasma pneumoniae Antibody on Colored Polystyrene Particles

The antibody prepared in Example 1 was diluted with purified water to a concentration of 1.0 mg/mL, and colored polystyrene particles were added thereto to a concentration of 0.1%. After stirring, carbodiimide was added to a concentration of 1%, and the resultant was further stirred. The supernatant was removed by centrifugation and resuspended in 50 mM Tris (pH 9.0), 3% BSA to obtain an anti-*Mycoplasma pneumoniae* antibody-bound colored polystyrene particles. In this example, it is referred to as an antibody-immobilized particles.

### 3. Preparation of Test Strips for Measuring Mycoplasma pneumoniae P1, P30 and P1 + P30

The antibody-immobilized membranes prepared in section 1 were attached to another members (the backing sheet, the absorption band, the sample pad) and cut to a width of 5 mm to prepare *Mycoplasma pneumoniae* test strips (see FIG. 2). These are referred to as test strips in this example.

### Example 4, Comparative Example 1 and Comparative Example 2

### Comparison of Sensitivities of Immunoassay Instruments for Measuring P1, P30 and P1 + P30

Three kinds of the P1 test strip (Comparative Example 1), the P30 test strip (Comparative Example 2) and the P1-P30 test strip (Example 4) were prepared according to the procedure of Example 3 from the combinations of the monoclonal antibodies shown in Table 2, which monoclonal antibodies were obtained in Example 1.

**[Table 2]**

| Test Strip | Antibody used | |
|---|---|---|
| | Antibody-immobilized membrane | Antibody-immobilized particles |
| P1 test strip (Comparative Example 1) | P1A | P1B |
| P30 test strip (Comparative Example 2) | P30A | P30B |
| P1-P30 test strip (Example 4) | P1-P30A | P1-P30A |

To each test strip, 50 µL of a sample suspension containing an arbitrarily diluted *Mycoplasma pneumoniae* antigen and the antibody-immobilized particles prepared in section 2 was added dropwise, and the resultant was allowed to stand for 15 minutes.

Each test sample was judged to be "+" when the color development was able to be visually confirmed at the application positions of both the anti-mouse IgG antibody and the anti-*Mycoplasma pneumoniae* antibody. Each test sample was judged to be "-" when the color development was able to be visually confirmed only at the application position of the anti-mouse IgG antibody and the color development was not able to be visually confirmed at the application position of the anti-*Mycoplasma pneumoniae* antibody. Each test sample was judged invalid when the color development was not able to be visually confirmed at the application position of the anti-mouse IgG antibody.

The results of each test strip are shown in Table 3.

**[Table 3]**

| | Blank | x32 | x64 | x128 | x256 | x512 | x1024 |
|---|---|---|---|---|---|---|---|
| P1 test strip (Comparative Example 1) | - | + | - | - | - | - | - |
| P30 test strip (Comparative Example 2) | - | + | + | ± | - | - | - |
| P1-P30 test strip (Example 4) | - | + | + | + | + | + | ± |

As shown in Table 3, the immunoassay instrument of the present invention for measuring both P1 and P30 of *Mycoplasma pneumoniae* showed 16 times higher sensitivity compared with the immunoassay instrument for measuring P1 alone and 8 times higher sensitivity compared with the immunoassay instrument for measuring P30 alone.

### Example 5

The antibodies used in the P1-P30 test strip were tested in the combination of P1-P30A and P1-P30B in the same manner as in Example 4. As a result, they showed 2 to 4 times higher sensitivity compared with the combination of only P1-P30A (Example 4).

### DESCRIPTION OF THE SYMBOLS

- 1: support
- 2: labeled substance region
- 3: detection region
- 4: sample pad
- 5: absorption band
- 6: backing sheet

## Claims

1. A method of detecting *Mycoplasma pneumoniae* having two or more kinds of antigens which enable detection of said *Mycoplasma pneumoniae,*
said method comprising detecting said *Mycoplasma pneumoniae* by an immunoassay using a monoclonal antibody or an antigen-binding fragment thereof which recognizes said two or more kinds of antigens,
wherein said two or more kinds of antigens are P1 protein and P30 protein.

2. The method according to claim 1, wherein said immunoassay is a sandwich method in which said monoclonal antibody or an antigen-binding fragment thereof is used in at least one of a label and a solid phase.

3. The method according to claim 2, wherein said immunoassay is an immunochromatography method.

4. The method according to any one of claims 1 to 3, wherein said two or more kinds of antigens recognized by said monoclonal antibody form a composite.

5. An immunoassay instrument for carrying out the method according to claim 1 for detecting *Mycoplasma pneumoniae,*
wherein said monoclonal antibody or an antigen-binding fragment thereof which recognizes said two or more kinds of antigens which are P1 protein and P30 protein is used in at least one of a label and a solid phase.

6. The immunoassay instrument according to claim 5, wherein said immunoassay instrument is an immunochromatographic strip.
***

7. A monoclonal antibody specifically reacting with P1 protein and P30 protein derived from *Mycoplasma pneumoniae.*

## Patentansprüche

1. Verfahren zum Detektieren von *Mycoplasma pneumoniae* mit zwei oder mehr Antigenarten, die die Detektion von *Mycoplasma pneumoniae* ermöglichen,
wobei das Verfahren das Detektieren von *Mycoplasma pneumoniae* mittels eines Immunassays unter Verwendung eines monoklonalen Antikörpers oder eines antigenbindenden Fragments davon umfasst, der/das die zwei oder mehr Antigenarten erkennt,
wobei die zwei oder mehr Antigenarten P1-Protein und P30-Protein sind.

2. Verfahren nach Anspruch 1, wobei das Immunassay ein Sandwichverfahren ist, bei dem der monoklonale Antikörper oder das antigenbindende Fragment davon in zumindest einem aus einer Markierung und einer festen Phase verwendet wird.

3. Verfahren nach Anspruch 2, wobei das Immunassay ein Immunchromatographie-Verfahren ist.

4. Verfahren nach einem der Ansprüche 1 bis 3, wobei die zwei oder mehr Antigenarten, die von dem monoklonalen Antikörper erkannt werden, einen Verbund bilden.

5. Immunassay-Instrument zur Durchführung eines Verfahrens nach Anspruch 1 zum Detektieren von *Mycoplasma pneumoniae,*
wobei der monoklonale Antikörper oder das antigenbindende Fragment davon, der/die die zwei oder mehr Antigenarten erkennt, bei denen es sich um P1-Protein und P30-Protein handelt, in zumindest einem aus einer Markierung und einer festen Phase verwendet wird.

6. Immunassay-Instrument nach Anspruch 5, wobei das Immunassay-Instrument ein Immunchromatographiestreifen ist.

7. Monoklonaler Antikörper, der spezifisch mit von *Mycoplasma pneumoniae* abgeleitetem P1-Protein und P30-Protein reagiert.

## Revendications

1. Procédé de détection de *Mycoplasma pneumoniae* ayant deux ou plus de deux types d'antigènes qui permettent une détection de ladite *Mycoplasma pneumoniae*,
ledit procédé comprenant la détection de ladite *Mycoplasma pneumoniae* par un immunodosage utilisant un anticorps monoclonal ou un fragment de liaison à l'antigène de celui-ci qui reconnaît lesdits deux ou plus de deux types d'antigènes,
dans lequel lesdits deux ou plus de deux types antigènes sont la protéine P1 et la protéine P30.

2. Procédé selon la revendication 1, dans lequel ledit immunodosage est un procédé sandwich dans lequel ledit anticorps monoclonal ou un fragment de liaison à l'antigène de celui-ci est utilisé dans au moins l'un parmi un marqueur et une phase solide.

3. Procédé selon la revendication 2, dans lequel ledit immunodosage est un procédé d'immunochromatographie.

4. Procédé selon l'une quelconque des revendications 1 à 3, dans lequel lesdits deux ou plus de deux types d'antigènes reconnus par ledit anticorps monoclonal forment un composite.

5. Instrument d'immunodosage pour la mise en œuvre du procédé selon la revendication 1 de détection de *Mycoplasma pneumoniae*,
dans lequel ledit anticorps monoclonal ou un fragment de liaison à l'antigène de celui-ci qui reconnaît lesdits deux ou plus de deux types d'antigènes qui sont la protéine P1 et la protéine P30 est utilisé dans au moins l'un parmi un marqueur et une phase solide.

6. Instrument d'immunodosage selon la revendication 5, dans lequel ledit instrument d'immunodosage est une bandelette immunochromatographique.

7. Anticorps monoclonal réagissant spécifiquement avec la protéine P1 et la protéine P30 dérivées de *Mycoplasma pneumoniae.*
